# EUROPEAN PATENT SPECIFICATION

(11) **EP 3 677 233 B1**
(45) Date of publication and mention of the grant of the patent: **05.01.2022**
(21) Application number: 18852087.8
(22) Date of filing: 30.08.2018
(51) Int. Cl.: A61F 13/511, A61F 13/47

(54) **ABSORBENT ARTICLE**
ABSORBIERENDER ARTIKEL
ARTICLE ABSORBANT

(30) Priority: 30.08.2017 JP 2017165874
(43) Date of publication of application: 08.07.2020
(73) Proprietor: Daio Paper Corporation, Ehime 799-0492 (JP)
(72) Inventor: ODA, Terumitsu, Sakura-shi Tochigi 329-1411 (JP)
(74) Representative: Epping - Hermann - Fischer
(86) International application number: PCT/JP2018/032107
(87) International publication number: WO 2019/044967

(56) References cited:
- WO-A1-2015/146717
- JP-A- 2008 136 563
- JP-A- 2008 136 563
- JP-A- 2011 200 445
- JP-A- 2015 073 775
- JP-A- 2016 097 107
- JP-A- 2016 199 830

## Description

### Technical Field

The present invention relates to an absorbent article such as a sanitary napkin, a panty liner, and an incontinence pad, and more particularly to an absorbent article, in which a plurality of embossed portions recessed to a non-skin side are formed in a given pattern on a surface of a skin side of a top sheet.

### Background Art

Conventionally, as an absorbent article, an absorbent article made of a cotton-like pulp or the like, which is interposed between a liquid impermeable back sheet such as a polyethylene sheet or a polyethylene laminate non-woven fabric and a liquid permeable top sheet such as a non-woven fabric or a liquid-permeable plastic sheet, is known.

As a result of continuous improvements, and an embossed portion recessed toward a non-skin side is provided on a skin side surface of the liquid permeable top sheet in various patterns for the purpose of improving an absorption performance of a body fluid. For example, Patent Document 1 discloses a three-dimensional shape non-woven fabric that includes irregularities on at least one surface, linear pressure-bonding parts on bottom portions of recessed parts, to which constituent fibers are crimped or bonded, and a large number of projected parts surrounded by the recessed parts, in which a large number of closed regions surrounded by the pressure-bonding parts are formed. Also, Patent Document 2 discloses an absorbent article, in which a dot-like embossed pattern is imparted to a top sheet in a regular pattern.

Patent Document 3 relates to a surface sheet for an absorbent article having a heat-shrinkable fiber layer containing heat-shrinkable heat-shrinkable fibers and having a plurality of embossed portions. The top sheet has a plurality of large polygonal regions surrounded by the plurality of embossed portions, and the embossed portions form the tops of the large polygonal regions. The surface sheet has a plurality of small polygonal areas that are surrounded by the embossed parts that form the tops of the plurality of large polygonal areas and have a smaller area than the large polygonal areas, and the embossed parts are the small polygonal areas. The top of the. High convex portions are formed in each of the large polygon regions, and low convex portions having a height lower than that of the high convex portions are formed in each of the small polygon regions. A plurality of large polygon regions are arranged adjacent to each other along the first direction, and a plurality of small polygon regions are arranged adjacent to each other along the first direction. The small polygonal region rows configured as described above are alternately arranged in a second direction orthogonal to the first direction.

### [Related-Art Documents]

### Patent Documents

[Patent Document 1] Japanese Unexamined Patent Application Publication No. 2011-137262
[Patent Document 2] Japanese Unexamined Patent Application Publication No. 2008-136563
[Patent Document 3] International Patent Application Publication No. WO 2015/146717 A1

### Summary of the Invention

### Problem to be Solved by the Invention

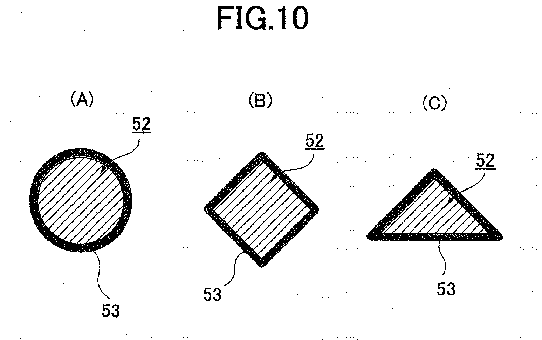

However, the conventional absorbent articles described in patent documents 1 and 2 have the following problems.
(1) As depicted in FIG. 8, in a case in which continuous linear embossed portions 50 described in Patent Document 1 are formed, a diffusion of a body fluid is accelerated along the continuous linear embossed portions 50, so that a diffusion range of the body fluid having conspicuous color such as menstrual blood is increased, and there is a possibility of causing an unpleasant feeling to a user viewing the used absorbent article (problem of liquid spreading property).
(2) In a case of the continuous linear embossed portions 50 described in Patent Document 1 and regularly arranged dot-like embossed portions 51 of Patent Document 2, as depicted in FIG. 8 and FIG. 9, high-density regions of fiber are formed around the embossed portions 50 and 51 by the squeezing, the body fluid absorbed in a periphery by capillary action is attracted to the high-density regions around the embossed portions, and liquid is held in void parts between the fibers, thereby forming a liquid intensive region 53 along the periphery of the embossed portions; however, the body fluid aggregated in the liquid intensive region 53 is conspicuous and thus, an appearance of an used absorbent article is deteriorated (problem of liquid intensive property). In particular, in the case of the embossed portions 50 each formed in a linear or dot-like shape, because the liquid intensive regions 53 formed along both sides of the linear embossed portions 50 and the periphery of the dot-like embossed portions 51 are close to each other, even though they are separated by the embossed portions 50 and 51, the whole of the adjacent liquid intensive regions 53 exhibits a more conspicuous appearance such as one line or one large lump.
(3) In order to make the body fluid aggregated around the embossed portions inconspicuous in appearance, as depicted in FIG. 10, an area of an embossed portion 52 is increased to increase a separation width of the liquid intensive region 53 formed along the periphery of the embossed portion 52 so as not to be visually integrated; however, since the area of the embossed portion 52 is increased, the touch feeling is deteriorated (problem of touch feeling).

However, in the absorbent article described in Patent Document 2, as a planar shape of each dot-like embossed portion, because an outline connecting the protruding parts is formed by a broken line (straight line) that bulges inward, when the absorbent article is attached, a top sheet is easily bent with a bent part (intersection of straight lines) as a base point, and accompanying this, a corner part due to the bending of the top sheet is formed, resulting in a cause of a tingling and unpleasant touch.

A main object of the present invention is to provide an absorbent article in which an absorbed body fluid is made inconspicuous in appearance without deteriorating a touch feeling.

### Means to Solve the Problem

According to a first aspect of the present invention in order to solve the above described problem, an absorbent article, in which an absorbent body is interposed between a liquid permeable top sheet and a liquid impermeable back sheet, includes
a plurality of embossed portions arranged at intervals on a skin side surface of the liquid permeable top sheet, each embossed portion recessed on a non-skin side,
wherein each embossed portion is formed in a planar shape passing through each corner of an original shape and having concave shaped outlines each formed by a curve bulging each side of the original shape toward a center, in which a polygon having four or more sides is defined as the original shape.

Further, corners of the original shape of adjacent embossed portions are arranged in a direction facing each other with respect to a longitudinal direction of the absorbent article, and are arranged in a staggered pattern shifted by a half pitch in the longitudinal direction of every other row with respect to a width direction of the absorbent article.

In the present invention claimed as claim 1, on the skin side of the liquid permeable top sheet, the plurality of embossed portions depressed on the non-skin side are arranged at intervals; therefore, as compared with a case in which a continuous linear embossed portion is formed, the diffusion range of the body fluid becomes smaller, and the absorbed body fluid becomes inconspicuous in appearance.

Moreover, the embossed portion is formed in a planar shape passing through each corner of the original shape, having the concave shaped outlines each formed by the curve passing through respective corners of the original shape, and bulging each side of the original shape toward the center, in which the polygon having four or more sides is defined as the original shape, and liquid intensive regions formed along peripheries of the embossed portions are close to corners of the original shape and a vicinity of the original shape; however, the liquid intensive regions are separated at a center of the original shape, whereby similar to a linear or dot-shaped embossed portion, the entire liquid intensive regions sandwiching the embossed portion are not integrated and conspicuous.

Moreover, in the embossed portion according to the present invention, because each perimeter of the embossed portions is increased by the concave shaped outlines bulging toward the center of the original shape while reducing a compressed area, it is possible to reduce the amount of the body fluid contained per unit length of the liquid intensive region, and the body fluid is dispersed so that the effect of making the color of the body fluid less noticeable is also exerted.

Furthermore, because each outline of the embossed portion is formed in a concave shape formed by a curved line, it is difficult to form a bending base point of the liquid permeable top sheet on the outline, and it is possible to prevent the touch feeling from becoming worse.

According to the present invention, in the absorbent article according to any one of the first to third aspects, the adjacent embossed portions are arranged so that corners of the original shape face each other.

According to the present invention, the plurality of embossed portions are arranged in such a manner that corners of the original shape of adjacent embossed portions face each other in a case in which the plurality of embossed portions are arranged. That is, the embossed portions are arranged such that ends of portions extending to the corners of the original shape face each other. By this arrangement, on each line connecting the ends of the portions extending to the corners of the original shape between the adjacent embossed portions, an influence line of the squeezing, in which the fiber is slightly densified, is formed due to an effect of the squeezing of each embossed portion, so that the body fluid is less likely to be diffused outside the influence line, and the body fluid is more likely to be retained in a region surrounded by the embossed portions and the influence line between the embossed portions. For this reason, the diffusion range of the body fluid becomes smaller, and the absorbed body fluid becomes less noticeable.

According to a second aspect of the present invention, in the absorbent article according to the first aspect, the original shape is formed by a square.

According to the second aspect of the present invention, the embossed portion has a substantially cross-shaped planar shape by making the original shape a quadrangle, and as compared to a case of forming a pentagon or more, an angle formed by the outlines on both sides of a part extending to respective corners of the original shape and a separation distance between the parts extending to respective corners of the original shape are able to be relatively increased, whereby the liquid intensive region is able to be made less noticeable.

According to a third aspect of the present invention, in the absorbent article according to the first or second aspect, each of sides of the original shape is 2-20 mm in length.

According to the third aspect of the present invention, the liquid intensive region formed along the periphery of the outline may be closer and more noticeable when a length of each side of the original shape is smaller than 2 mm, and the compressed area of the embossed portion 10 increases when the length of each side is larger than 20 mm, so that the touch feeling may be deteriorated; therefore, a length of one side of the original shape is 2 to 20 mm.

According to a fourth aspect of the present invention, in the absorbent article according to any one of the first to third aspects, a size of each embossed portion is larger than an interval between adjacent embossed portions.

According to the fourth aspect of the present invention, the size of each embossed portion is larger than the interval between the adjacent embossed portions, whereby the body fluid is easily concentrated around the embossed portion, the body fluid is easily held in the region surrounded by the embossed portion, the diffusion area of the body fluid is reduced, and the absorbed body fluid becomes less noticeable.

According to a fifth aspect of the present invention, in the absorbent article according to any one of the first to fourth aspects, an interval between adjacent embossed portions is 2 to 20 mm

In the fifth aspect of the present invention, the body fluid easily flows along the embossed portions when the interval between the adjacent embossed portions is smaller than 2 mm, and the influence line of the squeezing is hardly formed over the entire length between the adjacent embossed portions when the interval is larger than 20 mm and thus, the body fluid is easily diffused outside the region surrounded by the embossed portions and the influence line between the embossed portions, so that the interval between the adjacent embossed portions is set to 2 to 20 mm.

According to a seventh aspect of the present invention, in the absorbent article according to any one of the first to sixth aspects, ends of the outlines extending to respective corners of the original shape are intersected or are chamfered in a curved line or a straight line.

In the seventh aspect of the present invention, by intersecting the ends of the outlines extending to the respective corners of the original shape, or by chamfering the outlines in a curved or straight line, between the adjacent embossed portions, the fiber density of the influence line of the squeezing is able to be adjusted, and a degree of diffusion of body fluid is able to be controlled to some extent.

According to an eighth aspect of the present invention, in the absorbent article according to any one of the first to seventh aspects, at least an inside of a circle with a diameter of 1 mm inscribed in each of the embossed portions is squeezed.

In the eighth aspect of the present invention, in order to secure a compressed portion at a central portion of each embossed portion, at least the inside of the circle with a diameter of 1 mm inscribed in each of the embossed portions is squeezed.

### Effects of the Invention

As described in detail above, according to the present invention, an absorbed body fluid becomes inconspicuous in appearance without deteriorating the touch feeling.

### Brief Description of the Drawings

FIG. 1 is a partially exploded development view of a sanitary napkin 1 according to the present invention.
FIG. 2 is a view taken along line II-II of FIG. 1.
FIG. 3 is a planer view of an embossed portion 10.
FIG. 4 is a planer view illustrating a diffusion state of a body fluid around an embossed portion 10.
FIG. 5(A) and FIG. 5(B) are planer views of an embossed portion 10 according to a modification.
FIG. 6 is a planer view illustrating an arrangement pattern of embossed portions 10 according to a modification.
FIG. 7(A) and FIG. 7(B) are enlarged views of ends of an outline 14 extending to respective corners 12 of the embossed portion 10.
FIG. 8 is a planer view (part 1) illustrating a diffusion state of a body fluid in a conventional embossed portion 50.
FIG. 9 is a planer view (part 2) illustrating the diffusion state of the body fluid in the conventional embossed portion 50.
FIG. 10 is a planer view (part 3) illustrating the diffusion state of the body fluid in the conventional embossed portion 50.

### Mode for Carrying out the Invention

In the following, embodiments of the present invention are described below with reference to the accompanying drawings.

### [Basic Constitution of Sanitary Napkin 1]

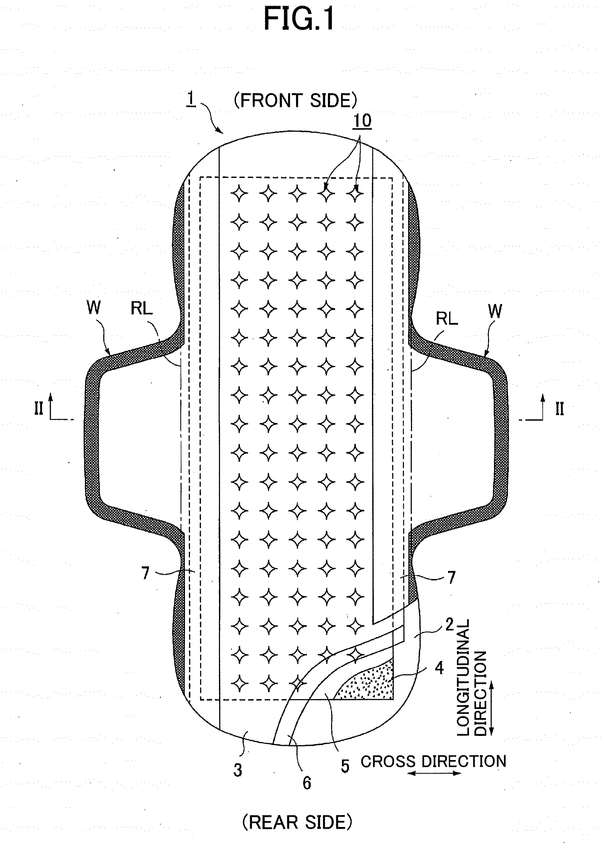

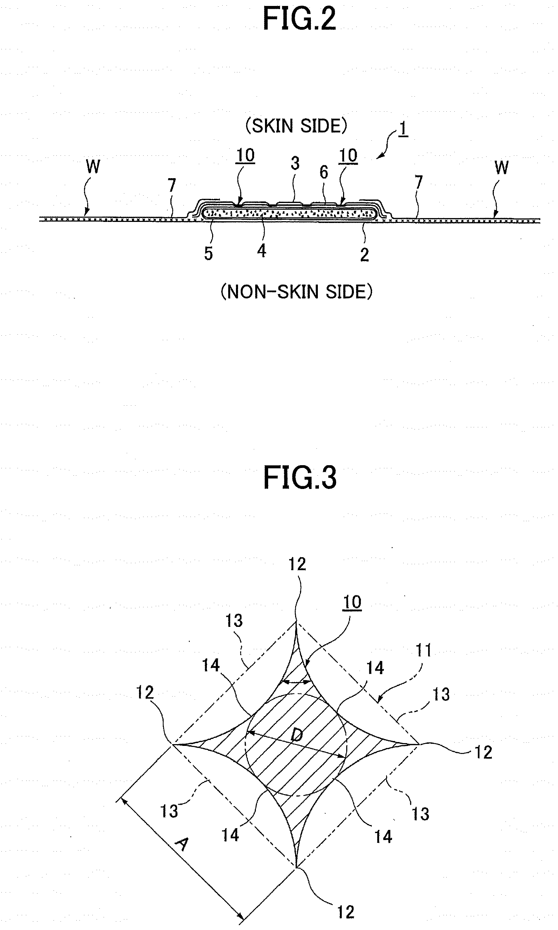

As described in FIG. 1 and FIG. 2, a sanitary napkin 1 of the present invention includes a liquid impermeable back sheet 2 made of a polyethylene sheet, a polypropylene sheet, and the like, a liquid permeable top sheet 3 for rapidly transmitting menstrual blood or the like, an absorbent body 4 made of cotton-like pulp or synthetic pulp interposed between both sheets 2 and 3, a wrapping sheet 5 made of a crepe paper or non-woven fabric surrounding the absorbent body 4 in order to improve a shape retention and a diffusion property of the absorbent body 4, a hydrophilic second sheet 6 arranged between the liquid permeable top sheet 3 and the absorbent body 4 as necessary, and side sheets 7, 7 arranged along the longitudinal direction on both side parts on a skin contact surface side. At a periphery of the absorbent body 4, in predetermined regions at both edges in a longitudinal direction of a napkin, the liquid impermeable back sheet 2 and the liquid permeable top sheet 3 are bonded to each other by an adhesive such as a hot-melt adhesive or an adhesive such as a heat seal or an ultrasonic seal, and the liquid impermeable back sheet 2 and the side sheets 7, which are extended to the sides from the edges of the absorbent body 4 at the both edges of the napkin, are bonded to each other by an adhesive such as the hot-melt adhesive or the adhesive such as the heat seal or the ultrasonic seal, whereby an outer peripheral flap part, in which the absorbent body 4 does not exist on an outer periphery.

The structure of the sanitary napkin 1 will be described in detail below.

For the liquid impermeable back sheet 2, a sheet material having at least a water-shielding property such as an olefin-based resin sheet such as polyethylene or polypropylene is used; alternatively, a laminated non-woven fabric sheet, in which a non-woven fabric is laminated on a polyethylene sheet or the like; furthermore, a non-woven fabric sheet or the like may be used by interposing a waterproof film to substantially ensure liquid impermeability (in this case, a liquid permeable top sheet is formed by a waterproof film and non-woven fabric). In recent years, moisture permeability tends to be used from a viewpoint of prevention of stuffiness. This moisture permeable sheet material is a microporous sheet, which is obtained by stretching a sheet in an uniaxial or biaxial direction after the sheet is formed by melt-kneading an inorganic filler in an olefinic resin such as polyethylene or polypropylene.

A non-woven fabric is preferably used for the liquid permeable top sheet 3. As the material fiber forming the non-woven fabric, for example, synthetic fibers such as olefins such as polyethylene or polypropylene, polyesters, polyamides, the like, recycled fibers such as rayon and cupra, and natural fibers such as cotton may be used, and a non-woven fabric, which is obtained by a suitable processing method such as a spun lace method, a spun bonding method, a thermal bonding method, a melt-blown method, a needle punching method, may be used. Among these processing methods, the spun lace method is excellent in flexibility, the spun bonding method is excellent in drapeability, and the thermal bond method and the air-through method are excellent in that they are bulky and have high compression restorability. Also, composite fibers, such as core-sheath type fibers with high melting point fiber as core and low melting point fiber as sheath, side-by-side type fibers and split type fibers, may be used. In order to achieve with a side sheet 7, it is preferable to mix synthetic fibers for the liquid permeable top sheet 3.

As the absorbent body 4, which is interposed between the liquid impermeable back sheet 2 and the liquid permeable top sheet 3, for example, a material obtained by mixing a highly water-absorbing resin into pulp or a material obtained by mixing a chemical fiber into a pulp and also mixing a highly water-absorbing resin is used. As depicted in the figure, it is desirable that the absorbent body 4 is surrounded by the wrapping sheet 5 in order to maintain the shape and quickly diffuse menstrual blood and the like, and prevent the menstrual blood and the like once absorbed from returning. Examples of the pulp include those made of cellulose fibers such as chemical pulp and molten pulp obtained from wood, and artificial cellulose fibers such as rayon and acetate; and softwood pulp having a longer fiber length than hardwood pulp is more preferably used in terms of function and price. Moreover, as the absorbent body 4, an air-laid absorbent body whose bulk may be reduced, or a polymer sheet, in which a superabsorbent resin is arranged between two non-woven fabric layers, may be used.

Also, synthetic fibers may be mixed in the absorbent body 4. For the synthetic fibers, for example, polyolefins such as polyethylene or polypropylene, polyesters such as polyethylene terephthalate or polybutylene terephthalate, polyamides such as nylon, and copolymers thereof, and the like may be used, and a mixture of these two types may be used. Moreover, composite fibers such as a core-sheath type fiber having a high melting point fiber as a core and a low melting point fiber as a sheath, a side-by-side type fiber, and a split type fiber, or the like may be used. As the synthetic fiber, in a case of a hydrophobic fiber, it is desirable to use a surface-treated synthetic fiber having a hydrophilicity agent so as to have an affinity for a body fluid.

Examples of the superabsorbent resin include a crosslinked product of polyacrylate, a crosslinked product of self-crosslinked polyacrylate, a saponified product of a crosslinked product of an acrylate-vinyl acetate copolymer, a crosslinked product of an isobutylene/maleic anhydride copolymer, cross-linked polysulfonate, partially crosslinked water-swellable polymers such as polyethylene oxide and polyacrylamide, and the like. Among these examples, acrylic acid or acrylate-based compounds having an excellent water absorption and an excellent water absorption speed are preferred. In a production process of a superabsorbent resin having the water absorbing performance, a water absorbing ability and the water absorbing speed are able to be adjusted by adjusting a crosslinking density and a crosslinking density gradient.

In a case in which the wrapping sheet 5 surrounding the absorbent body 4 is provided as in these examples, the wrapping sheet 5 is interposed between the liquid permeable top sheet 3 and the absorbent body 4 as a result. In a case in which the wrapping sheet 5 is made of crepe paper, the wrapping sheet 5 having excellent absorptivity allows the body fluid to be quickly diffused and prevents the menstrual blood from returning.

The second sheet 6 may be any kind as long as it has hydrophilicity to body fluids. Specifically, a material itself including hydrophilicity may be used by using regenerated fibers such as rayon and cupra, and natural fibers such as cotton; alternatively, a fiber, which has hydrophilic property imparted by surface-treating a synthetic fiber such as olefins such as polyethylene or polypropylene, polyesters, polyamides, or the like with a hydrophilic agent, may be used. In addition, the second sheet 6 may have a porous film layer on a back surface side (absorbent body 4) in order to have a firmness, may be a laminated sheet with an enveloped sheet, and further may be a material containing pulp.

On both sides of the sanitary napkin 1 on the skin contact surface side, side sheets 7, 7 are respectively provided along a longitudinal direction and over substantially the entire length of the napkin 1, side sheets 7, 7 are extended laterally, and the liquid impermeable back sheet 2 also extended laterally, whereby flap portions are formed on both sides of a main body portion where the absorbent body 4 is not interposed. Wing-shaped flaps W, W protruding outward are formed by parts of the side sheets 7 and parts of the liquid impermeable back sheet 2, which extend laterally. The wing-shaped flaps W, W are used by being folded outward at a fold line RL (FIG. 1) at a base end portion so as to involve a crotch portion of an underwear at a time of wearing, and slip-resistant adhesive layers (not shown), which are provided on an outer surface of the liquid impermeable back sheet 2 of the wing-shaped flaps W, are attached to an outer surface of the underwear, so that slippage from the underwear is prevented.

As the side sheets 7, a water-repellent non-woven fabric or a hydrophilic non-woven fabric may be used from a viewpoint of a function regarded as important. For example, if emphasis is placed on functions such as preventing menstrual blood or vaginal discharge from penetrating or enhancing the feel of the skin, it is desirable to use a water-repellent non-woven fabric coated with a silicon-based, paraffin-based, alkylchromic chloride-based water-repellent, or the like. Also, if emphasis is placed on the absorbability of menstrual blood and the like in the wing-shaped flaps W, W, it is desirable to use a hydrophilically-treated nonwoven fabric that has been rendered hydrophilic by applying capillary action, which the synthetic fibers are made to be swollen or porous by a method of polymerizing in the presence of a compound having a hydrophilic group, for example, an oxidation product of polyethylene glycol or the like in the process of producing a synthetic fiber, a method of treating with a metal salt such as stannic chloride, partially dissolving a surface to form porous and depositing a metal hydroxide. It is preferable that a synthetic fiber is blended in the side sheets 7 in order to achieve fusion with the liquid permeable top sheet 3.

### [Embossed Portions 10]

As depicted in FIG. 1, a plurality of embossed portions 10, 10, ... depressed toward the non-skin side are arranged at predetermined intervals on a skin side surface (outer surface) of the liquid permeable top sheet 3. Each of the embossed portions 10 is a concave shaped portion which is integrally recessed from the liquid permeable top sheet 3 to the second sheet 6 by squeezing from the outer surface side of the liquid permeable top sheet 3.

As depicted in FIG. 3, a planar shape of each of the embossed portions 10 is a polygon having four or more sides, that is in the illustrated example, a square having four sides is used as an original shape 11, and each of the embossed portions 10 is formed in a shape having concave shaped outlines 14 each formed by a curve passing through respective corners 12 of the original shape 11 and bulging each side 13 of the original shape 11 toward the center. By this configuration, a planar shape of each of the embossed portions 10 is formed by a central portion composed of a squeezed portion that is relatively wide in a portion including a center of the original shape 11, and a plurality of radial portions formed with a plurality of compressed portions relatively narrower than the central portion such that the compressed portion are formed continuously from the center in a tapered shape extending radially toward each of corners 12 of the original shape 11.

The original shape 11 is represented by a virtual figure used to draw each of the embossed portions 10 and is not arranged on an actual sanitary napkin 1 as depicted in FIG. 1. The original shape 11 is able to be recognized by connecting ends of adjacent radial portions of one embossed portion 10 with straight lines.

In each of the embossed portions 10, a region (shaded region depicted in FIG. 3) surrounded by outlines 14, 14, ... is depressed toward the non-skin side, and constituent fibers are crimped at a bottom. At the periphery thereof, although not directly compressed, a high-density region of the fiber, in which the fiber is slightly densified, is formed due to an influence of squeezing such as a drawing of the fiber or the like when forming the embossed portion 10.

The embossed portion 10 is formed, in which a total length (perimeter) of the outlines 14, 14,... is formed longer than a total length (perimeter) of sides 13, 13, ... of the original shape 11, and an area is smaller than an area of the original shape 11.

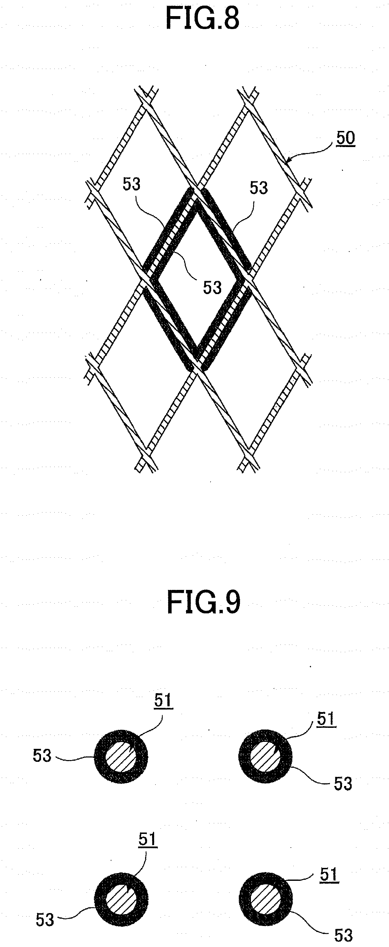

Because the plurality of the embossed portions 10 are formed at intervals in the sanitary napkin 1, as depicted in FIG. 8, compared with a case in which conventional continuous linear embossed portions 50 are formed, flows of body fluid along the embossed portions 10 do not occur so that a diffusion range of the body fluid becomes small, and an absorbed body fluid becomes inconspicuous in appearance. That is, in a case of the conventional linear embossed portions 50, the body fluid flows in concave grooves of the embossed portions 50 and the body fluid also diffuses in the high-density regions of the fiber formed around the embossed portions 50 by the influence of the pressing of the embossed portions 50, whereby flows of body fluids along the embossed portions 50 are likely to occur; however, because the embossed portions 10 according to the present invention are arranged at predetermined intervals, the flows and diffusions of these body fluids are suppressed.

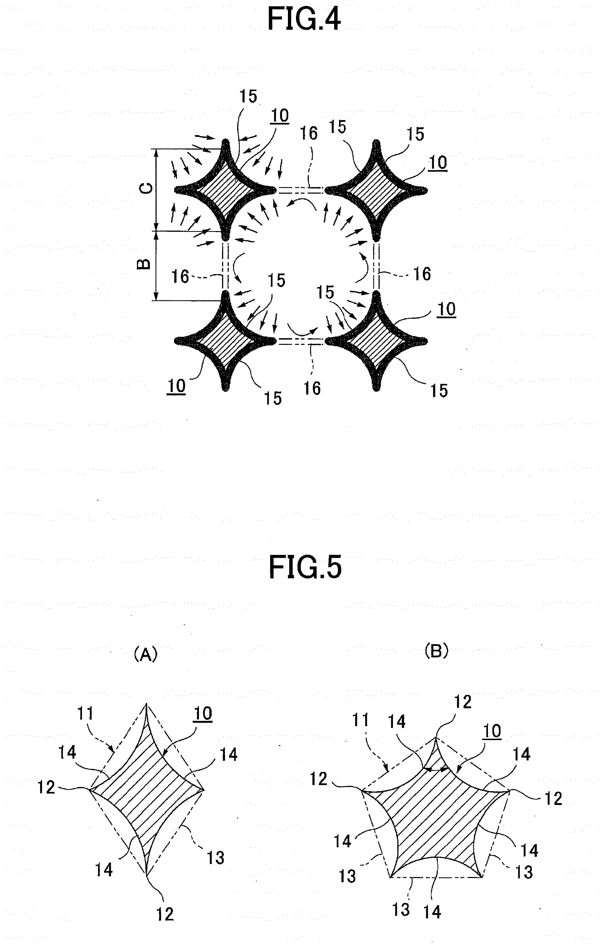

Also, because each of the embossed portions 10 is formed in the planar shape having concave shaped outlines 14 shaping curves each obtained by bulging each side 13 of the original shape 11 toward the center, in which a polygon having the number of four or more sides is defined as the original shape 11 and passes through each corner 12 of the original shape 11, as depicted in FIG. 4, the absorbed body fluid is less noticeable from the viewpoint of the liquid intensity of the embossed portions 10. Specifically, at the periphery of each of the embossed portions 10, the high-density region of fibers is formed along with the outline 14 of the embossed portion 10 due to squeezing when forming the embossed portion 10. The high-density region of the fibers becomes a liquid intensive region 15, in which the body fluid is held in gaps between the fibers by the body fluid absorbed into the periphery of the embossed portion 10 by a capillary action. A liquid intensive region 15 is formed along the periphery of the concave shaped outline 14 of each of the embossed portions 10. In the embossed portion 10 according to the present invention, each of the liquid intensive regions 15, 15 on both sides is close to each other at ends of the radial portions extending to the corners 12 of the original shape 11 and vicinities thereof; however, because the liquid intensive regions 15 are separated at a central portion other than the ends and the vicinities, and at neighboring portions of the central portions, the liquid intensive regions 15, 15 adjacent to each other are less likely to be integrated on the external appearance, and the absorbed body fluid is less visible on the external appearance.

Also, because as compared with the original shape 11, each perimeter of the embossed portions 10 is increased by the concave shaped outlines 14 bulging toward the center of the original shape 11 while reducing a compressed area (seal area), the amount of the body fluid contained per unit length of the liquid intensive region 15 is able to be reduced, and an effect of dispersing the body fluid makes a color of the body fluid less visible in appearance.

Moreover, each of the embossed portions 10 is formed in a planar shape having a concave shaped outline 14 formed by a curve obtained by bulging each side 13 of the original shape 11 toward the center, the compressed area (seal area) of the embossed portion 10 is reduced so that it is possible to prevent the touch feeling from becoming worse, as compared with a case in which the original shape 11 is compressed to form the embossed portion.

Furthermore, because each of the outlines 14 of the embossed portion 10 is formed in a concave shape forming a curve, it is difficult to form a bending base point of the liquid permeable top sheet 3 on the outline 14, and it is possible to prevent the touch feeling from being deteriorated.

Also, By forming the outline 14 by a curve, if a maximum separation distance (depth of bulging toward the center side) from the original shape 11 is the same, forming with a curve is able to make an outline longer than forming with a broken line (straight line), the liquid intensive region 15 becomes less noticeable in appearance.

Each of the outlines 14 of the embossed portion 10 is preferably formed by a curve bulging toward the center of the original shape 11, particularly an arc. The arc is a part of a circumference formed with a constant radius. It is preferable that each outline 14 and each side 13 have a maximum separation width at the central portion, and are formed so that the separation width gradually decreases toward both ends (corners 12). Each of the outlines 14, 14, ... is extended between adjacent corners 12, 12 of the original shape 11 without intersecting each other.

In a case in which the original shape 11 is a rhombus obtained by rotating a regular square by 45 degrees with respect to the longitudinal direction of the sanitary napkin 1, and the outline 14 is the arc as depicted in FIG. 1 and FIG. 3, the planar shape of the embossed portion 10 has radial portions projecting from the central portion in four directions of the longitudinal direction and a width direction of the sanitary napkin 1, respectively as depicted in FIG. 1 and FIG. 3, so as to be formed in a substantially cross shape formed by a curve, in which both side edges of the radial portion bulge inward. Moreover, as a modified example, as depicted in FIG. 5(A), the original shape 11 does not need to be formed with the same length in one direction and another direction in the longitudinal direction and the width direction of the sanitary napkin 1, and may be formed as the rhombus having a relatively long direction and a short direction. Furthermore, as depicted in FIG. 5(B), by forming the original shape 11 in a pentagonal shape, the planar shape of the embossed portion 10 may be formed in a substantially star shape. Although not depicted, the original shape 11 may be formed as a hexagon or a higher polygon.

In particular, it is desirable that the original shape 11 is formed in a quadrangle. By making the original shape 11 the quadrangle, the planar shape of the embossed portion 10 becomes a substantially cross shape and as depicted in FIG. 3 and FIG. 5(B), and an angle formed by the outlines 14 on both sides of a radial portion, which radially protrude, is relatively larger than a case in which the original shape 11 is formed as a pentagon or more, so that the liquid intensive regions 15 formed along the outlines 14 on both sides are no longer close to each other, and the liquid intensive regions 15 are able to be made less noticeable. Moreover, as a number of corners of the original shape 11 increases, an interval between adjacent radial portions decreases, and the liquid intensive regions 15 become more conspicuous. Therefore, it is preferable to form the original shape 11 with as few corners as possible. In a case in which the original shape 11 is a triangle, the perimeter is too short to disperse the body fluids collected in the liquid intensive region 15, so that the body fluids collected in the liquid intensive region 15 become more conspicuous.

As depicted in FIG. 3, a length A of one side of the original shape 11 (a length between adjacent corners 12, 12) is preferably 2 to 20 mm, particularly preferably 5 to 10 mm. The liquid intensive region 15 formed along the periphery of the outline 14 may be closer and more noticeable when the length A is smaller than 2 mm, and the compressed area of the embossed portion 10 increases when the length A is larger than 20 mm, so that the touch feeling may be deteriorated.

In a case in which the outline 14 is formed by an arc, a radius of the arc is preferably 1 to 20 mm, particularly preferably 3 to 10 mm. In a case in which the radius of the arc is smaller than 1 mm, a plurality of liquid intensive regions 15 may be conspicuous in close proximity. Moreover, in a case in which the radius of the arc is larger than 20 mm, the width of swelling from each side 13 of the original shape 11 to the center side is small and the compressed area of the embossed portion 10 becomes large, so that the touch feeling may be deteriorated.

The embossed portion 10 is preferably formed such that at least a 1 mm diameter, and an inside of a circle having preferably a 1 mm to 10 mm diameter is squeezed from the center. In other words, as depicted in FIG. 3, when drawing a circle (dash-dotted line) in contact with ends of the outlines 14, 14 ... bulging to respective insides, a diameter D of an inscribed circle may be 1 mm or more. Thus, each of the liquid intensive regions 15 formed along the periphery of the embossed portion 10 has a predetermined separation width at the center of the embossed portion 10, so that the liquid intensive regions 15 are able to be reliably made inconspicuous in appearance.

A planar arrangement pattern of the plurality of embossed portions 10, 10, ... may be any pattern, and as depicted in FIG. 1 and FIG. 4, it is preferable in the adjacent embossed portions 10 that the corners 12 of the original shape 11 are arranged so as to face each other. In a case in which the embossed portion 10 is formed in a substantially cross shape, it is preferable as depicted in FIG. 1 that the plurality of embossed portions 10, 10,... are preferably arranged in a regular lattice shape aligned in the longitudinal direction and the width direction of the sanitary napkin 1. By arranging the corners 12 of the original shape 11 in opposite directions, as depicted in FIG. 4, between the adjacent embossed portions 10, 10, ... on a line connecting the ends of the radial portions extending to the corners 12 of the original shape 11, an influence line 16 of the squeezing, in which the fibers are slightly densified, is formed due to the influence of the squeezing of each embossed portion 10, whereby the body fluid is less likely to be diffused outside the influence line 16, and the body fluid is more likely to be retained in a region surrounded by the embossed portions 10, 10, ... and the influence lines 16, 16, .... Therefore, the diffusion range of the body fluid becomes small, and the absorbed body fluid becomes less noticeable.

As depicted in FIG. 4, an interval B between the adjacent embossed portions 10, 10 is preferably 2 to 20 mm. In a case in which the interval B between the embossed portions 10, 10 is smaller than 2 mm, the body fluid easily flows along the separated embossed portions 10. In a case in which the interval B is larger than 20 mm, it is difficult for the influence line 16 of the squeezing to be formed over the entire length between the adjacent embossed portions 10 and 10, and the body fluid is more likely to diffuse outside the region surrounded by the embossed portions 10, ... and the influence lines 16 between the embossed portions 10, ....

As depicted in FIG. 4, it is preferable that a size C of the embossed portion 10 (length of the largest portion of a planar dimension of the embossed portion 10) is larger than the interval B between the adjacent embossed portions 10, 10. Therefore, the body fluid is easily collected in the liquid intensive region 15 around the embossed portion 10, and the body fluid is easily retained in the region surrounded by the embossed portions 10, 10, whereby a diffusion area of the body fluid becomes smaller, and the absorbed body fluid becomes less noticeable.

Moreover, by making the size C of the embossed portion 10 larger than the interval B between the embossed portions 10, 10, it becomes difficult for the liquid permeable top sheet 3 to bend from a part of the embossed portion 10 on each outline 14 as a base point, and the effect of forming the outline 14 by a curve is able to be promoted.

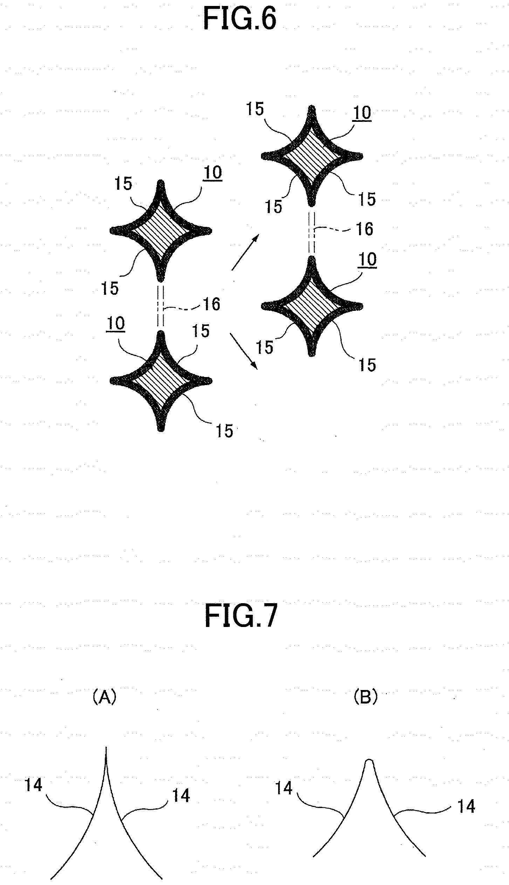

As a modified example of the arrangement pattern of the embossed portions 10, as depicted in FIG. 6, the corners 12 of the original shape 11 of the adjacent embossed portions 10, 10 may be arranged in a direction facing each other with respect to the longitudinal direction of the sanitary napkin 1, and may be arranged in a staggered pattern shifted by a half pitch in the longitudinal direction of every other row with respect to the width direction of the sanitary napkin 1. By this arrangement, between the embossed portions 10, 10 ... aligned in the longitudinal direction of the sanitary napkin 1, the influence line 16 of the squeezing is easily formed between the adjacent embossed portions 10, 10, and diffusion of the body fluid in the width direction is suppressed; however, because the embossed portions 10, 10 are not arranged facing each other in the width direction of the sanitary napkin 1, the influence line 16 along the width direction of the sanitary napkin 1 is not formed, and the body fluid easily spreads in the longitudinal direction of the sanitary napkin 1 through a space between the embossed portions 10 and 10. Thus, it is possible to prevent leakage of the body fluid from both sides.

As depicted in FIG. 7, it is preferable that the ends of the outlines 14 on both sides of the radial portion extending to respective corners 12 of the original shape 11 intersect or be chamfered in a curved or straight line. FIG. 7(A) is a cross-section of the ends of the outlines 14, and FIG. 7(B) is a chamfer in a curved shape. By the shape of this end portion, it is possible to adjust the fiber density of the influence line 16 of the squeezing between the adjacent embossed portions 10 and 10, and it is possible to control the degree of the diffusion of the body fluid. In particular, as depicted in FIG. 7(A), in a case in which the ends of the outlines 14 are intersected with each other, a shape of the ends becomes acute, so that the influence line 16 of the squeezing is narrow, and the effect of suppressing the diffusion of the body fluid is reduced. Also, as depicted in FIG. 7(B), in a case in which the ends are chamfered in the curved shape, the influence line 16 of the squeezing is slightly thicker than in a case in which the ends form an acute angle, and the effect of suppressing the diffusion of the body fluid is slightly improved. Moreover, although not depicted, in a case in which the ends are straight chamfered, the influence line 16 of the squeezing becomes further thicker than in the case in which the ends are chamfered to form the curved shape, and thus, it is possible to improve the effect of suppressing the diffusion of body fluid. In a case of providing the curved chamfer, it is preferable to use an arc line (R=3) having a radius of 3 mm or less, and in a case of providing the straight chamfer, the length of the chamfer is preferably 3 mm or less.

### Description of the Reference Numeral

1 sanitary napkin, 2 liquid impermeable back sheet, 3 liquid permeable top sheet, 4 absorbent body, 5 wrapping sheet, 6 second sheet, 7 side sheet, 10 embossed portion, 11 original shape, 12 corner, 13 side, 14 outline, 15 liquid intensive region, and 16 influence line

## Claims

1. An absorbent article (1), in which an absorbent body (4) is interposed between a liquid permeable top sheet (3) and a liquid impermeable back sheet (2), the absorbent article (1) comprising:
a plurality of embossed portions (10) arranged at intervals on a skin side surface of the liquid permeable top sheet (3), each embossed portion (10) recessed on a non-skin side,
wherein each embossed portion (10) is formed in a planar shape passing through each corner (12) of an original shape (11) and having concave shaped outlines (14) each formed by a curve bulging each side of the original shape (11) toward a center, in which a polygon having four or more sides is defined as the original shape (11),
wherein corners (12) of the original shape (11) of adjacent embossed portions (10) are arranged in a direction facing each other with respect to a longitudinal direction of the absorbent article (1), and are arranged in a staggered pattern shifted by a half pitch in the longitudinal direction of every other row with respect to a width direction of the absorbent article (1).

2. The absorbent article (1) as claimed in claim 1, wherein the original shape (11) is formed by a square.

3. The absorbent article (1) as claimed in claim 1 or 2, wherein each of sides of the original shape (11) is 2-20 mm in length.

4. The absorbent article (1) as claimed in any one of claims 1 to 3, wherein a size of each embossed portion (10) is larger than an interval between adjacent embossed portions (10).

5. The absorbent article (1) as claimed in any one of claims 1 to 4, wherein an interval between adjacent embossed portions (10) is 2 to 20 mm.

6. The absorbent article (1) as claimed in any one of claims 1 to 5, wherein ends of the outlines extending to respective corners (12) of the original shape (11) are intersected or are chamfered in a curved line or a straight line.

7. The absorbent article (1) as claimed in any one of claims 1 to 6, wherein at least an inside of a circle with a diameter of 1 mm inscribed in each of the embossed portions (10) is squeezed.

## Patentansprüche

1. Absorbierender Artikel (1), bei dem ein absorbierender Körper (4) zwischen einen flüssigkeitsdurchlässigen oberseitigen Flächenkörper (3) und einen flüssigkeitsundurchlässigen rückseitigen Flächenkörper (2) gelegt ist, wobei der absorbierende Artikel (1) aufweist:
eine Vielzahl von Prägeabschnitten (10), die mit Abständen an einer Hautseitenoberfläche des flüssigkeitsdurchlässigen oberseitigen Flächenkörpers (3) angeordnet sind, wobei jeder Prägeabschnitt (10) an einer Nicht-Hautseite vertieft ist,
wobei jeder Prägeabschnitt (10) in einer planaren Form ausgebildet ist, die durch jede Ecke (12) einer ursprünglichen Form (11) verläuft und konkav ausgebildete Umrisse (14) hat, die jeweils durch eine Krümmung gebildet sind, die sich an jeder Seite der ursprünglichen Form (11) zu einer Mitte hin wölbt, bei dem ein Polygon mit vier oder mehr Seiten als die ursprüngliche Form (11) definiert ist,
wobei Ecken (12) der ursprünglichen Form (11) von benachbarten Prägeabschnitten (10) in einer Richtung angeordnet sind, in der sie in Bezug auf eine Längsrichtung des absorbierenden Artikels (1) einander zugewandt sind, und die in Bezug auf eine Breitenrichtung des absorbierenden Artikels (1) in einem versetzten Muster angeordnet sind, und zwar versetzt um einen halben Abstand in Längsrichtung jeder zweiten Reihe.

2. Absorbierender Artikel (1) nach Anspruch 1, wobei die ursprüngliche Form (11) durch ein Quadrat gebildet ist.

3. Absorbierender Artikel (1) nach Anspruch 1 oder 2, wobei jede Seite der ursprünglichen Form (11) 2 bis 20 mm lang ist.

4. Absorbierender Artikel (1) nach einem der Ansprüche 1 bis 3, wobei eine Größe jedes Prägeabschnitts (10) größer als ein Abstand zwischen benachbarten Prägeabschnitten (10) ist.

5. Absorbierender Artikel (1) nach einem der Ansprüche 1 bis 4, wobei ein Abstand zwischen benachbarten Prägeabschnitten (10) 2 bis 20 mm beträgt.

6. Absorbierender Artikel (1) nach einem der Ansprüche 1 bis 5, wobei Enden der Umrisse, die sich zu jeweiligen Ecken (12) der ursprünglichen Form (11) erstrecken, sich in einer gekrümmten Linie oder einer geraden Linie schneiden oder ausgekehlt sind.

7. Absorbierender Artikel (1) nach einem der Ansprüche 1 bis 6, wobei zumindest eine Innenseite eines Kreises mit einem Durchmesser von 1 mm, der in jeden der Prägeabschnitte (10) eingeschrieben ist, zusammengedrückt ist.

## Revendications

1. Article absorbant (1) dans lequel un corps absorbant (4) est intercalé entre une feuille supérieure (3) perméable aux liquides et une feuille arrière (2) imperméable aux liquides, l'article absorbant (1) comprenant :
une pluralité de parties bosselées (10) agencées à intervalles sur une surface côté peau de la feuille supérieure (3) perméable aux liquides, chaque partie bosselée (10) étant évidée d'un côté non peau,
sachant que chaque partie bosselée (10) est formée en une forme plane passant par chaque coin (12) d'une forme d'origine (11) et ayant des contours de forme concave (14) formés chacun par une courbe bombant chaque côté de la forme d'origine (11) vers un centre, dans laquelle un polygone à quatre côtés ou plus est défini comme la forme d'origine (11) ,
sachant que des coins (12) de la forme d'origine (11) de parties bosselées (10) adjacentes sont agencés dans une direction en face l'un de l'autre par rapport à une direction longitudinale de l'article absorbant (1), et sont agencés en un motif échelonné décalé d'un demi-pas dans la direction longitudinale d'une rangée sur deux par rapport à une direction de largeur de l'article absorbant (1).

2. L'article absorbant (1) tel que revendiqué dans la revendication 1, sachant que la forme d'origine (11) est formée par un carré.

3. L'article absorbant (1) tel que revendiqué dans la revendication 1 ou 2, sachant que chacun des côtés de la forme d'origine (11) a une longueur de 2 à 20 mm.

4. L'article absorbant (1) tel que revendiqué dans l'une quelconque des revendications 1 à 3, sachant qu'une taille de chaque partie bosselée (10) est plus grande qu'un intervalle entre des parties bosselées (10) adjacentes.

5. L'article absorbant (1) tel que revendiqué dans l'une quelconque des revendications 1 à 4, sachant qu'un intervalle entre des parties bosselées (10) adjacentes est de 2 à 20 mm.

6. L'article absorbant (1) tel que revendiqué dans l'une quelconque des revendications 1 à 5, sachant que des extrémités des contours s'étendant vers des coins (12) respectifs de la forme d'origine (11) sont croisés ou sont chanfreinés en une ligne courbe ou une ligne droite.

7. L'article absorbant (1) tel que revendiqué dans l'une quelconque des revendications 1 à 6, sachant qu'au moins un intérieur d'un cercle ayant un diamètre de 1 mm inscrit dans chacune des parties bosselées (10) est écrasé.
